# EUROPEAN PATENT APPLICATION

(11) **EP 2 944 696 A1**
(43) Date of publication of application: **18.11.2015**
(21) Application number: 14168130.4
(22) Date of filing: 13.05.2014
(51) Int. Cl.: C12P 7/02

(54) **Method of producing organic compounds**

(71) Applicant: Evonik Degussa GmbH, 45128 Essen (DE)
(72) Inventor: Haas, Thomas, 48161 Münster (DE); Demler, Martin, 46286 Dorsten (DE); Eckl, Eva-Maria, 45768 Marl (DE); Beck, Simon, 48163 Münster (DE)

(57) **Abstract**

There is provided a mixed culture of a first and second microorganism in an aqueous medium, wherein the first microorganism is an acetogenic microorganism capable of converting a carbon source comprising CO and/or CO₂ to acetate and/or ethanol, the second microorganism is a non-acetogenic microorganism capable of metabolising acetate and/or ethanol and the first and second microorganisms are in a homogenous mixture. There is also provided a method of producing substituted and/or unsubstituted organic compounds using the mixed culture.

## Description

### FIELD OF THE INVENTION

The present invention relates to a mixed culture of microorganisms, a method of using the mixed culture of microorganisms to produce organic compounds and a use of the mixed culture of microorganisms. In particular, the mixed culture of microorganisms comprises at least an acetogenic and a non-acetogenic microorganism.

### BACKGROUND OF THE INVENTION

The use of carbon dioxide as a carbon source for synthesis of organic compounds using a microbiological process is well known in the art. In general, the method comprises two complementary metabolic pathways one involving the production of acetate and/or ethanol and the other converting this acetate and/or ethanol to an organic compound. The methods known in the art try to combine these two complementary metabolic pathways from different organisms in a recombinant cell for production of the organic substance.

This raises several problems as each organism used in the method is specialised and requires specific conditions to function and carry out the individual metabolic reaction. Combining these organisms to produce organic compounds is thus a difficult task as the organisms are not as efficient as they will not be able to work at optimal conditions.

Accordingly, currently available methods of synthesising carbon sources to produce organic compounds use a two-step process where each organism is allowed to carry out its function in its optimal condition. These two-step processes usually use two chambers where each chamber has an organism in its optimal condition for carrying out the specific metabolic reaction and the product of the first chamber is transferred to the second chamber without the residual first organism. This process is cumbersome and usually more costly as a large part of the resultant product from the first chamber may be lost in the transfer process to the second chamber. This two-step process is also generally inefficient as it takes time to allow one metabolic pathway to be completed and transfer the first resultant product to the second chamber for the second metabolic pathway to be carried out. WO2013167663 and WO2013036147 both describe methods for production of specific hydrocarbons using a two-step process.

There are alternative methods known and used where CO₂ is used as a carbon source by a microorganism which is known to be able to fix CO₂, through a specific selection of the fermentation parameters , so that a desired , simple organic substance , such as ethanol, n-butanol or 2,3 -butanediol is synthesized. For example, WO200068407 describes the use of acetogenic bacteria to produce ethanol and WO2012024522 uses acetogenic bacteria for the production of butanediol.

However, most of the processes described have the disadvantage that the yields are low and the method is inefficient.

It is an object of the present invention to provide a process which can overcome at least one disadvantage of the prior art.

### DESCRIPTON OF THE INVENTION

The present invention provides a mixed cell culture, method and use of the culture as defined in the claims.

An advantage of the present invention may be that much more favorable CO₂/CO mixtures of raw materials can be used. These various sources include natural gas, biogas, coal, oil, plant residues and the like.

Another advantage of the method may be the high carbon yield. This is made possible by the return of formed CO₂. Namely, the CO₂ can be reacted in the first stage back to acetic acid. Another advantage may lie in greater flexibility with regard to the fermentation conditions used, as any acetogenic and non-acetogenic microorganism may be used in combination for the actual production of unsaturated or saturated organic compounds.

Another advantage of the present invention may be that since the acetogenic microorganism may function and/or produce acetate and/or ethanol in the presence of oxygen, both the acetogenic and non-acetogenic microorganisms may be present in a homogenous mixture for the production of substituted and/or unsubstituted organic compounds from a carbon source. This feature of the acetogenic microorganism enables the production of substituted and/or unsubstituted organic compounds from a carbon source to be a one step process making the process more efficient and the yield greater. Surprisingly, because of this advantage of the acetogenic microorganism, the one-step procedure for making organic compounds may be carried out in a single fermenter without an intermediate separation step. There may also be an increased concentration of the final product using this one step procedure.

According to a first aspect of the present invention, there is provided a mixed culture of a first and second microorganism in an aqueous medium, wherein
- the first microorganism may be an acetogenic microorganism and/or autotrophically growing cells capable of converting a carbon source comprising CO and/or CO₂ to acetate and/or ethanol;
- the second microorganism may be a non-acetogenic microorganism capable of metabolising acetate and/or ethanol; and
- the first and second microorganisms may be in a homogenous mixture.

The term 'homogeneous mixture' as used herein refers to a mixture of the microorganisms distributed uniformly in a medium. In particular, the mixture may comprise at least two microorganisms, an acetogenic and a non-acetogenic microorganism distributed evenly in a medium. In one example, there may be approximately equal numbers of acetogenic and non-acetogenic microorganisms in the mixture. In another example, there may be more of the acetogenic microorganism compared to the non-acetogenic microorganism in the mixture. In yet another example, there may be more of the non-acetogenic microorganism compared to the acetogenic microorganism in the mixture. In all the possible examples, the microorganisms are in a single homogenous mixture where they are uniformly distributed throughout the mixture.

The term "acetate" as used herein, refers to both acetic acid and salts thereof, which results inevitably, because as known in the art, since the microorganisms work in an aqueous environment, and there is always a balance between salt and acid present.

The term "second microorganism" refers to a microorganism that is different from "the first microorganism" according to any aspect of the present invention. Accordingly, the first and second microorganisms are different according to any aspect of the present invention.

According to a second aspect of the present invention, there is provided a method of producing at least one organic compound, the method comprising contacting a mixed culture of a first and second microorganism in a homogenous mixture in an aqueous medium, with a carbon source, wherein
- the first microorganism is an acetogenic microorganism capable of converting a carbon source to acetate and/or ethanol; and
- the second microorganism is a non-acetogenic microorganism capable of metabolising acetate and/or ethanol to the organic compound.

The aqueous medium according to any aspect of the present invention may comprise oxygen. The oxygen may be dissolved in the medium by any means known in the art. In particular, the oxygen may be present at a range of 0.1 to 3mg/l. In particular, the oxygen may be present at a range of the 0.1 to 2.5mg/l, 0.1 to 2 mg/l, 0.1 to 1.5 mg/l or 0.5 to 2.0 mg/l.

In particular, the oxygen may be provided to the aqueous medium in a continuous gas flow. The oxygen concentration is the gas flow may be present at less than 21 % by weight of the total amount of gas in the gas flow. In particular, the oxygen may be present at a concentration range of 0.01 to 10 % by weight, at a range of 0.05 to 10 % by weight, 0.1 to 10 % by weight, 0.1 to 9 % by weight, 0.05 to 8 % by weight, particularly at a range of 0.05 to 6 % by weight. In particular, the acetogenic microorganism is particularly suitable when the proportion of O₂ in the gas phase is between 0.05 % and 6 %. Even more in particular, the first and second microorganisms according to any aspect of the present invention are capable of working optimally in the aqueous medium when the oxygen is supplied by a gas flow with concentration of oxygen of less than 21 % by weight of the total gas, in the range of 0.05 to 6 % by weight.

The term "acetogenic microorganism" as used herein refers to a microorganism which is able to perform the Wood-Ljungdahl pathway and thus is able to convert CO, CO₂ and/or hydrogen to acetate. These microorganisms include microorganisms which in their wild-type form do not have a Wood-Ljungdahl pathway, but have acquired this trait as a result of genetic modification. The acetogenic microorganism may be any microorganism that may be autotrophically cultured. Examples include but are not limited to *Acetogenium kivui, Acetobacterium woodii, Acetoanaerobium noterae, Clostridium aceticum, Butyribacterium methylotrophicum, Clostridium acetobutylicum, Clostridium thermoaceticum, Eubacterium limosum, Peptostreptococcus productus, Clostridium Ijungdahlii,* and *Clostridium carboxydivorans.* In the same connection, the term 'non-acetogenic microorganism' refers to any microorganism which is incapable of performing the Wood-Ljungdahl pathway.

In particular, the term 'non-acetogenic microorganism' may refer to any non-acetogenic microorganism known in the art. More in particular, this term as used herein, refers to a microorganism which may fall within the category of an aerotolerant microorganism, facultative anaerobe, aerobic microorganisms and the like. The non-acetogenic bacteria may be selected from the group consisting of *Alpha sp., Perlucidibaca sp., Thiothrix sp., Janthinobacterium sp., Spongiibacter sp., Congregibacter sp., Salipiger sp., Thermobifida sp., Anoxybacillus sp., Agarivorans sp., Desmospora sp., Providencia sp., Lautropia sp., Deinococcus sp., Ralstonia sp., Proteus sp., Leucothrix sp., Thiomonas sp., Neisseria sp., Sphingopyxis sp., Sporosarcina sp., Cyclobacterium sp., Dokdonia sp., Blastomonas sp., Lutibaculum sp., Mesorhizobium sp., Betaproteobacteria sp., Halorhodospira sp., Novosphingobium sp., Sandarakinorhabdus sp., Sphingobium sp., Gammaproteobacteria sp., Marinomonas sp., Afipia sp., Rhodoferaxsp., Dechloromonas sp., Polaromonas sp., Ferrimonas sp., Anaeromyxobacter sp., Polynucleobacter sp., Psychromonas sp.,Acidovorax sp., Xenorhabdus sp. Nitrobacter sp., Oceanicaulis sp., Rubrobacter sp., Solibacter sp., Rhodopseudomonas sp., Chromohalobacter sp., Chelativorans sp., Burkholderia sp., Stenotrophomonas sp., Maricaulis sp., Alkalilimnicola sp., Verminephrobacter sp., Flavobacterium sp., Mycobacterium sp., Rhodospirillum sp., Escherichia sp., Pseudomonas sp., Xanthomonas sp., Rhodomicrobium sp., Bradyrhizobium sp., Acidiphilium sp., Sphingomonas sp., Acinetobacter sp., Salmonella sp., Micrococcus sp., Corynebacterium sp., Elizabethkingia sp., Spirochaeta sp., Agrobacterium sp., Klebsiella sp., Bordetella sp., Halomonas sp., Photorhabdus sp., Loktanella sp., Leucobacter sp., Cupriavidus sp., Shinella sp., Rubellimicrobium sp., Photobacterium sp., Vibrio sp., Shewanella sp., Caulobacter sp., Bacillus sp., Yersinia sp., Paenibacillus sp., Actinoplanes sp., Candidatus sp., Microbacterium sp., Nitrincola sp., Streptomyces sp., Castellaniella sp., Sinorhizobium sp., Pseudoalteromonas sp., Alteromonas sp., Thalassolituus sp., Hymenobacter sp., Paracoccus sp., Hydrogenophaga sp., Methylibium sp., Granulibacter sp., Aeromonas sp., Kutzneria sp., Nocardia sp., Advenella sp., Sphingomonas sp., Oleispira sp., Rhizobium sp., Brucella sp., Enterobacter sp. Alcanivorax sp., Clostridium sp., Variovorax sp., Psychrobacter sp., Serratia sp., Simiduia sp., Polaribacter sp., Ralstonia sp., Geobacillus sp., Thioalkalivibrio sp., Edwardsiella sp., Arthrobacter sp., Nocardiopsis sp., Gordonia sp., Oceanimonas sp., Rucella sp., Collimonas sp., Oligotropha sp., Achromobacter sp., Stigmatella sp., Pantoea sp., Herbaspirillum sp., Vlladdlia sp., Moraxella sp., Rhodobacter sp., Flavobacteriaceae sp., Shigella sp., Lysinibacillus sp., Janthinobacterium sp, Hyphomonas sp., Rhodococcus sp., Myxococcus sp., Hahella sp., Colwellia sp., Thermus sp., Chromobacterium sp., Dickey sp., Marinobacter sp., Chryseobacterium sp., Roseomonas sp., Xenorhabdus sp., Citrobacter sp., Saccharopolyspora sp., Herminiimonas sp., Frankia sp., Aromatoleum sp., Cycloclasticus sp., Laribacter sp., Brevibacillus sp., Erythrobacter sp., Rhodothermus sp., Rhodanobacter sp., Thiocystis sp., Sulfobacillus sp., Leifsonia sp., Oceanospirillales sp., Massilia sp., Rheinheimera sp., Moritella sp., Amphritea sp., Teredinibacter sp., Oxalobacteraceae sp., Dasani sp., Salinimonas sp., Marinobacterium sp., Ahrensia sp., Meganema sp., Oligella sp., Rubrivivax sp. Glaciecola sp., Lacinutrix sp., Microlunatus sp., Isoptericola sp., Delftia sp., Alicycliphilus sp., Marinithermus sp., Thermaerobacter sp., Marivirga sp., Cronobacter sp., Pandoraea sp., Microbulbifer sp., Kordiimonas sp., Porphyrobacter sp., Enterobacter sp., Modestobacter sp., Halobacillus sp., Azospirillum sp., Aliivibrio sp., Kocuria sp., Azoarcus sp., Pectobacterium sp., Oceanobacillus sp., Dermacoccus sp., Brevibacterium sp., Providencia sp., Cesiribacter sp., Bhargavaea sp., Acidocella sp., Cecembia sp., Kingella sp., Bradyrhizobiaceae sp., Brenneria sp., Bradyrhizobiaceae sp., Thermus sp., Brevundimonas sp., Asticcacaulis sp., Kosakonia sp., Kordia sp., Alishewanella sp., Reinekea sp., Oceanobacter sp., Oceanospirillum sp., Nitrococcus sp., Methylacidiphilum sp., Azotobacter sp., Parachlamydia sp., Thiorhodospira sp., Thiorhodococcus sp. Thiocapsa sp., Sorangium sp., Erwinia sp., Grimontia sp., Vibrionales sp., Microscilla sp., Alteromonadales sp., Bermanella sp.* and *Neptuniibacter sp..*

Some examples of non-acetogenic microorganisms may include but are not limited to *Burkholderia rhizoxinica, Stenotrophomonas maltophilia, Mycobacterium bovis, Rhodospirillum centenum, Rhodospirillum thermophilum, Escherichia coli, Pseudomonas aeruginosa, Xanthomonas arboricola, Burkholderia cenocepacia, Rhodomicrobium udaipurense, Acinetobacter baumannii, Streptomyces fulvissimus, Salmonella enterica, Pseudomonas bauzanensis, Micrococcus luteus, Pseudomonas pseudoalcaligenes, Burkholderia caribensis,* Bacterium Ec32 (GI:610510261), *Corynebacterium glycinophilum, Elizabethkingia anophelis, Agrobacterium tumefaciens, Bordetella holmesii, Photorhabdus luminescens, Loktanella hongkongensis, Klebsiella pneumoniae, Shinella zoogloeoides, Rubellimicrobium mesophilum, Photobacterium phosphoreum, Vibrio parahaemolyticus, Vibrio cholerae, Shewanella oneidensis, Caulobacter crescentus, Vibrio fischeri, Mycobacterium smegmatis, Corynebacterium glutamicum, Burkholderia pseudomallei, Bacillus thuringiensis, Streptomyces coelicolor, Bacillus cereus, Pseudomonas chloritidismutans, Salinisphaera shabanensis, Bordetella pertussis, Bradyrhizobium japonicum, Yersinia pestis, Acinetobacter pittii, Acinetobacter nosocomialis, Paenibacillus larvae, Pseudomonas stutzeri, Candidatus Competibacter, Candidatus Accumulibacter, Streptomyces albus, Castellaniella defragrans, Sinorhizobium meliloti, Pseudoalteromonas lipolytica, Thalassolituus oleivorans, Hymenobacter swuensis, Para coccus aminophilus, Granulibacter bethesdensis, Aeromonas hydrophila, Kutzneria albida, Nocardia nova, Advenella mimigardefordensis, Pseudomonas cichorii, Sphingomonas sanxanigenens, Sinorhizobium fredii, Oleispira antarctica, Brucella suis, Bacillus licheniformis, Burkholderia thailandensis, Dickeya dadantii, Candidatus contendobacter, Alteromonas macleodii, Clostridium stercorarium, Variovorax paradoxus, Rhizobium etli, Serratia plymuthica, Simiduia agarivorans, Pseudomonas protegens, Pseudomonas denitrificans, Ralstonia solanacearum, Mycobacterium liflandii, Thioalkalivibrio nitratireducens, Edwardsiella ictaluri, Nocardiopsis alba, Burkholderia cepacia, Mycobacterium massiliense, Pseudomonas fluorescens, Mycobacterium intracellulare, Gordonia polyisoprenivorans, Rucella pinnipedialis, Collimonas fungivorans, Cupriavidus necator, Corynebacterium resistens, Oligotropha carboxidovorans, Vibrio vulnificus, Pseudomonas putida, Achromobacter xylosoxidans, Stigmatella aurantiaca, Pantoea vagans, Herbaspirillum seropedicae, Acinetobacter oleivorans, Waddlia chondrophila, Moraxella catarrhalis, Bacillus megaterium, Rhodobacter capsulatus, Candidatus Puniceispirillum, Brucella microti, Flavobacteriaceae bacterium, Corynebacterium kroppenstedtii, Bacillus anthracis, Shewanella piezotolerans, Shigella boydii, Lysinibacillus sphaericus, Renibacterium salmoninarum, Yersinia pseudotuberculosis, Brucella ovis, Burkholderia mallei, Arthrobacter aurescens, Mycobacterium avium, Hyphomonas neptunium, Rhodococcus jostii, Myxococcus xanthus, Hahella chejuensis, Pseudomonas syringae, Colwellia psychrerythraea, Candidatus Pelagibacter, Mycobacterium tuberculosis, Thermus thermophiles, Chromobacterium violaceum, Brucella melitensis, Mycobacterium ulcerans, Mycobacterium abscessus, Mycobacterium chelonae, Mycobacterium xenopi, Mycobacterium kansasii, Xenorhabdus cabanillasii, Xenorhabdus szentirmaii, Vibrio nigripulchritudo, Cupriavidus metallidurans, Sphingomonas wittichii, Acidiphilium cryptum, Flavobacterium johnsoniae, Pseudomonas mendocina, Mycobacterium gilvum, Polynucleobacter necessaries, Burkholderia vietnamiensis, Methylibium petroleiphilum, Verminephrobacter eiseniae, Polaromonas naphthalenivorans, Acidovorax citrulli, Marinobacter aquaeolei, Psychromonas ingrahamii, Shewanella amazonensis, Paracoccus denitrificans, Candidatus Ruthia, Solibacter usitatus, Rhodopseudomonas palustris, Burkholderia ambifaria, Maricaulis maris, Shewanella frigidimarina, Alkalilimnicola ehrlichii, Pseudoalteromonas atlantica, Rubrobacter xylanophilus, Sphingopyxis alaskensis, Candidatus Koribacter, Psychrobacter cryohalolentis, Nitrobacter hamburgensis, Chromohalobacter salexigens, Shewanella denitrificans, Burkholderia xenovorans, Rhodoferax ferrireducens, Novosphingobium aromaticivorans, Anaeromyxobacter dehalogenans, Nitrobacter winogradskyi, Ralstonia eutropha, Thermobifida fusca, Dechloromonas aromatic, Psychrobacter arcticus, Marinomonas ushuaiensis, Rhodococcus rhodochrous, Acidovorax avenae, Photorhabdus temperate, Janthinobacterium agaricidamnosum, Enterobacter cloacae, Ferrimonas balearica, Gammaproteobacteria bacterium, Clostridium ultunense, Alpha proteobacterium, Shewanella putrefaciens, Shewanella loihica, Halorhodospira halophile, Congregibacter litoralis, Mesorhizobium loti, Pseudomonas caeni, Spongiibacter tropicus, Perlucidibaca piscinae, Leucothrix mucor, Sandarakinorhabdus limnophila, Sphingopyxis baekryungensis, Halomonas anticariensis, Pseudomonas pelagia, Pseudoalteromonas ruthenica, Rhodococcus erythropolis, Proteus hauseri, Enterobacter aerogenes, Betaproteobacteria bacterium, Lutibaculum baratangense, Deinococcus proteolyticus, Acinetobacter tjernbergiae, Vibrio cyclitrophicus, Ralstonia pickettii, Dokdonia donghaensis, Psychrobacter aquaticus, Cyclobacterium qasimii, Sporosarcina newyorkensis, Enterobacter hormaechei, Neisseria bacilliformis, Rhodococcus equi, Lautropia mirabilis, Corynebacterium genitalium, Chryseobacterium gleum, Roseomonas cervicalis, Mycobacterium parascrofulaceum, Acinetobacter haemolyticus, Achromobacter piechaudii, Serratia odorifera, Corynebacterium jeikeium, Corynebacterium efficiens, Proteus mirabilis, Corynebacterium lipophiloflavum, Providencia stuartii, Pseudoalteromonas undina, Pseudoalteromonas spongiae, Pseudoalteromonas rubra, Pseudoalteromonas piscicida, Pseudoalteromonas marina, Pseudoalteromonas citrea, Pseudoalteromonas arctica, Pseudomonas alcaligenes, Mycobacterium canettii, Streptomyces venezuelae, Marinobacter hydrocarbonoclasticus, Xanthomonas citri, Xanthomonas axonopodis, Halomonas elongate, Xenorhabdus nematophila, Xenorhabdus bovienii, Citrobacter rodentium, Escherichia fergusonii, Saccharopolyspora erythraea, Herminiimonas arsenicoxydans, Frankia alni, Pseudoalteromonas haloplanktis, Aromatoleum aromaticum, Pantoea ananatis, Neisseria wadsworthii, Candidatus burkholderia, Sphingobium ummariense, Novosphingobium lindaniclasticum, Sphingobium lactosutens, Sphingobium quisquiliarum, Sphingobium baderi, Agarivorans albus, Thiothrix nivea, Salipiger mucosus, Mycobacterium yongonense, Laribacter hongkongensis, Rhodococcus opacus, Brevibacillus brevis, Erythrobacter litoralis, Xanthomonas campestris, Dechloromonas aromatic, Geobacillus kaustophilus, Thiocystis violascens, Sulfobacillus acidophilus, Geobacillus thermoglucosidasius, Leifsonia rubra, Sphingobium chinhatense, Vibrio splendidus, Cycloclasticus pugetii, Oceanospirillales bacterium, Gamma proteobacterium, Massilia niastensis, Rheinheimera perlucida, Psychrobacter lutiphocae, Oceanimonas smirnovii, Moritella marina, Amphritea japonica, Teredinibacter turnerae, Oxalobacteraceae bacterium, Dasania marina, Moraxella boevrei, Sphingomonas melonis, Pseudomonas thermotolerans, Colwellia piezophila, Halomonas lutea, Salinimonas chungwhensis, Marinobacterium rhizophilum, Polaribacter franzmannii, Halomonas zhanjiangensis, Pseudomonas alcaliphila, Ahrensia kielensis, Meganema perideroedes, Oligella ureolytica, Marinobacter lipolyticus, Sphingobium xenophagum, Mycobacterium hassiacum, Bradyrhizobium elkanii, Burkholderia pyrrocinia, Vibrio breoganii, Vibrio tasmaniensis, Vibrio crassostreae, Vibrio kanaloae, Halomonas smyrnensis, Salmonella bongori, Marinomonas posidonica, Marinomonas mediterranea, Deinococcus maricopensis, Yersinia enterocolitica, Rubrivivax gelatinosus, Flavobacterium indicum, Glaciecola nitratireducens, Microlunatus phosphovorus, Sphingobium chlorophenolicum, Isoptericola variabilis, Pseudomonas fulva, Alicycliphilus denitrificans, Marinithermus hydrothermalis, Acidiphilium multivorum, Arthrobacter phenanthrenivorans, Thermus scotoductus, Thermaerobacter marianensis, Marivirga tractuosa, Cronobacter turicensis, Arthrobacter arilaitensis, Pseudomonas luteola, Microbulbifer variabilis, Hahella ganghwensis, Kordiimonas gwangyangensis, Oligella urethralis, Cupriavidus taiwanensis, Vibrio genomosp., Vibrio rumoiensis, Leucobacter salsicius, Halomonas stevensii, Microbacterium yannicii, Halomonas jeotgali, Bordetella bronchiseptica, Enterobacter radicincitans, Modestobacter marinus, Halobacillus halophilus, Shewanella baltica, Marinobacter adhaerens, Flavobacterium branchiophilum, Corynebacterium variabile, Mycobacterium africanum, Streptomyces davawensis, Sphingobium japonicum, Photorhabdus asymbiotica, Aliivibrio salmonicida, Burkholderia multivorans, Kocuria rhizophila, Candidatus Solibacter, Rhizobium leguminosarum, Alcanivorax borkumensis, Pseudomonas entomophila, Bacillus clausii, Pectobacterium atrosepticum, Bordetella parapertussis, Oceanobacillus iheyensis, Brevibacterium mcbrellneri, Acinetobacter radioresistens, Corynebacterium lipophiloflavum, Corynebacterium amycolatum. Citrobacter youngae, Providencia rettgeri, Enterobacter cancerogenus, Providencia rustigianii, Providencia alcalifaciens, Cronobacter malonaticus, Cronobacter dublinensis, Cronobacter universalis, Cronobacter sakazakii, Cronobacter condiment, Xanthomonas translucens, Bacillus atrophaeus, Moraxella macacae, Pseudoalteromonas flavipulchra, Cesiribacter andamanensis, Mesorhizobium metallidurans, Marinobacter santoriniensis, Bhargavaea cecembensis, Glaciecola agarilytica, Glaciecola chathamensis, Glaciecola lipolytica, Alcanivorax hongdengensis, Alcanivorax pacificus, Rhizobium mesoamericanum, Thermaerobacter subterraneus, Glaciecola arctica, Glaciecola mesophila, Glaciecola polaris, Glaciecola psychrophila, Pseudoalteromonas luteoviolacea, Anoxybacillus flavithermus, Shigella flexneri, Shigella sonnei, Mycobacterium rhodesiae, Glaciecola pallidula, Pseudoalteromonas agarivorans, Marinobacter nanhaiticus, Geobacillus thermoglucosidans, Bacillus methanolicus, Pseudomonas savastanoi, Rheinheimera nanhaiensis, Lysinibacillus fusiformis, Halomonas elongate, Cecembia lonarensis, Bacillus isronensis, Paenibacillus alvei, Kingella kingae, Pseudomonas chlororaphis, Bradyrhizobiaceae bacterium, Brevundimonas diminuta, Asticcacaulis biprosthecum, Streptomyces clavuligerus, Kosakonia radicincitans, Escherichia albertii, Kordia algicida, Vibrio harveyi, marine gamma proteobacterium, Glaciecola punicea, Alishewanella agri, Shigella dysenteriae, Pseudomonas synxantha, Burkholderia terrae, Sphingobium indicum, Vibrio alginolyticus, Vibrio angustum, Photobacterium profundum, Nitrococcus mobilis, Oceanicaulis alexandrii, Methylacidiphilum fumariolicum, Azotobacter vinelandii, Flavobacterium frigoris, Pantoea stewartii, Parachlamydia acanthamoebae, Alishewanella jeotgali, Pseudomonas psychrotolerans, Photobacterium angustum, Marinobacter manganoxydans, Thiorhodospira sibirica, Neisseria shayeganii, Ralstonia syzygii,* blood disease bacterium, *Thiorhodococcus drewsii, Thiocapsa marina, Brevibacillus laterosporus, Xanthomonas gardneri, Xanthomonas periorans, Xanthomonas vesicatoria, Corynebacterium urealyticum, Bordetella petrii, Sorangium cellulosum, Brucella inopinata, Erwinia billingiae, Photobacterium damselae, Grimontia hollisae, Vibrio mimicus, Vibrio orientalis, Vibrio furnissii, Vibrio metschnikovii, Vibrio coralliilyticus, Azoarcus aromaticum, Erwinia carotovora, Vibrio albensis, Bordetella bronchiseptica, Shewanella benthica, Vibrio campbellii, Vibrionales bacterium, Microscilla marina, Alteromonadales bacterium, Rhodococcus fascians, Bermanella marisrubri, Neptuniibacter caesariensis, Pseudoalteromonas tunicate, Reinekea blandensis, Rubellimicrobium thermophilum, Halomonas boliviensis* and the like. In particular, the non-acetogenic bacteria may be selected from the group consisting of *Corynebacterium glutamicum, Halomonas boliviensis, Escherichia coli, Cupriavidus necator,* and *Pseudomonas putida.*

The term "aerobic microorganism" as used herein, refers to a microorganism which requires aerobic conditions (i.e.in the presence of oxygen) to live and grow. "Aerobic conditions" refers to concentrations of oxygen in the culture medium that are sufficient for an aerobic microorganism to use as a terminal electron acceptor. Such microorganisms include but are not limited to *E. coli, Corynebacterium glutamicum, Halomonas boliviensis, Pseudomonas putida* cells.

The term 'aerotolerant microorganism' as used herein, refers to aerotolerant anaerobic bacteria which are specialized anaerobic bacteria characterized by a fermentative-type of metabolism. These bacteria live by fermentation alone, regardless of the presence of oxygen in their environment. In particular, aerotolerant microorganisms or anaerobes cannot use oxygen for growth, but tolerate its presence. Exemplary aerotolerant anaerobic bacteria include but are not limited to *Desulfomonas, Butyrivibrio, Eubacterium, Lactobacillus, Clostridium, Ruminococcus, Cupriavidus necator and Propionibacterium.*

The term 'facultative anaerobe' as used herein refers to an organism that makes ATP by aerobic respiration if oxygen is present, but is capable of switching to fermentation or anaerobic respiration if oxygen is absent. Some examples of facultatively anaerobic bacteria are *Staphylococcus sp., Streptococcus sp., Escherichia coli, Listeria sp.* and *Shewanella oneidensis.* Certain eukaryotes are also facultative anaerobes, including fungi such as *Saccharomyces cerevisiae* and many aquatic invertebrates such as Nereid (worm) polychaetes.

The homogeneous mixture according to any aspect of the present invention may have a mixture of cultures. In one example, the homogenous mixture may comprise at least one acetogenic organism, and at least aerobic organism. In another example, the mixture may comprise at least one acetogenic microorganism and an aerotolerant organism or an acetogenic microorganism, an aerobic organism and an aerotolerant organism and the like. A skilled person would be capable of varying the types of cells in the homogenous mixture to obtain the best yield.

All percentages (%) are, unless otherwise specified, mass percent.

With respect to the source of substrates comprising carbon dioxide and/or carbon monoxide, a skilled person would understand that many possible sources for the provision of CO and/or CO₂ as a carbon source exist. It can be seen that in practice, as the carbon source of the present invention any gas or any gas mixture can be used which is able to supply the microorganisms with sufficient amounts of carbon, so that acetate and/or ethanol, may be formed from the source of CO and/or CO₂.

Generally for the mixed culture of the present invention the carbon source comprises at least 50 % by weight, at least 70 % by weight, particularly at least 90 % by weight of CO₂ and / or CO, wherein the percentages by weight-% relate to all carbon sources that are available to the first microorganism in the mixed culture.

In the mixed culture according to any aspect of the present invention, the carbon material source may be provided. Examples of carbon sources in gas forms include exhaust gases such as synthesis gas, flue gas and petroleum refinery gases produced by yeast fermentation or clostridial fermentation. These exhaust gases are formed from the gasification of cellulose-containing materials or coal gasification.

In one example, these exhaust gases may not necessarily be produced as by-products of other processes but can specifically be produced for use with the mixed culture of the present invention.

According to any aspect of the present invention, the carbon source may be synthesis gas. Synthesis gas can for example be produced as a by-product of coal gasification. Accordingly, the microorganism of the mixed culture according to any aspect of the present invention may be capable of converting a substance which is a waste product into a valuable resource.

In another example, synthesis gas may be a by-product of gasification of widely available, low-cost agricultural raw materials for use with the mixed culture of the present invention to produce substituted and unsubstituted organic compounds.

There are numerous examples of raw materials that can be converted into synthesis gas, as almost all forms of vegetation can be used for this purpose. In particular, raw materials are selected from the group consisting of perennial grasses such as miscanthus, corn residues, processing waste such as sawdust and the like.

In general, synthesis gas may be obtained in a gasification apparatus of dried biomass, mainly through pyrolysis, partial oxidation and steam reforming, wherein the primary products of the synthesis gas are CO, H₂ and CO₂.

Usually, a portion of the synthesis gas obtained from the gasification process is first processed in order to optimize product yields, and to avoid formation of tar. Cracking of the undesired tar and CO in the synthesis gas may be carried out using lime and/or dolomite. These processes are described in detail in for example, Reed, 1981.

Mixtures of sources can be used as a carbon source.

According to any aspect of the present invention, a reducing agent, for example hydrogen may be supplied together with the carbon source. In particular, this hydrogen may be supplied when the C and/or CO₂ is supplied and/or used. In one example, the hydrogen gas is part of the synthesis gas present according to any aspect of the present invention. In another example, where the hydrogen gas in the synthesis gas is insufficient for the method of the present invention, additional hydrogen gas may be supplied.

A skilled person would understand the other conditions necessary to carry out the method of the present invention. In particular, the conditions in the container (e.g. fermenter) may be varied depending on the first and second microorganisms used. The varying of the conditions to be suitable for the optimal functioning of the microorganisms is within the knowledge of a skilled person.

In one example, the method of the present invention may be carried out in an aqueous medium with a pH between 5 and 8, 5.5 and 7. The pressure may be between 1 and 10 bar.

In particular, the aqueous medium may comprise oxygen and a carbon source comprising CO and/or CO₂. More in particular, the oxygen and a carbon source comprising CO and/or CO₂ is provided to the aqueous medium in a continuous gas flow. Even more in particular, the continuous gas flow comprises synthesis gas and oxygen. In one example, both gases are part of the same flow/stream. In another example, each gas is a separate flow/stream provided to the aqueous medium. These gases may be divided for example using separate nozzles that open up into the aqueous medium, frits, membranes within the pipe supplying the gas into the aqueous medium and the like.

In one example according to any aspect of the present invention, the synthesis gas may be blended with the oxygen gas before being supplied into the aqueous medium. This blending step may improve the efficiency and the production of organic compounds in the reaction. The overall efficiency, alcohol productivity and/or overall carbon capture of the method of the present invention may be dependent on the stoichiometry of the CO₂, CO, H₂ and O₂ in the continuous gas flow. The continuous gas flows applied may be of composition O₂, CO₂ and H₂. In particular, in the continuous gas flow, concentration range of O₂ may be within 0.05 to 6 % by weight, CO₂ about 10-50 %, in particular 33 % by weight and H₂ would be within 44 % to 84 %, in particular, 64 to 66.04 % by weight. More in particular, the concentration of gases in the continuous gas flow may be 4 % by weight of O₂, 32 % by weight of CO₂ and 64 % by weight of H₂. In another example, the continuous gas flow can also comprise inert gases like N₂, up to a N₂ concentration of 50 % by weight.

A skilled person would understand that it may be necessary to monitor the composition and flow rates of the streams prior to blending. Control of the composition of the blended stream can be achieved by varying the proportions of the constituent streams to achieve a target or desirable composition. The composition and flow rate of the blended stream can be monitored by any means known in the art. In one example, the system is adapted to continuously monitor the flow rates and compositions of at least two streams and combine them to produce a single blended substrate stream in a continuous gas flow of optimal composition, and means for passing the optimised substrate stream to the mixed culture of the present invention.

In particular, the mixture of the first microorganism, the acetogenic organism, the second microorganism, an acetic acid or ethanol aerobically metabolizing organism , a gas mixture of oxygen and either carbon monoxide and or a mixture of carbon dioxide and hydrogen can be employed in any known bioreactor or fermenter to carry out any aspect of the present invention.

Microorganisms which convert CO₂ and/or CO to acetate and/or ethanol, in particular acetate, as well as appropriate procedures and process conditions for carrying out this metabolic reaction is well known in the art. Such processes are, for example described in WO9800558, WO2000014052, WO2010115054, Demler et al., Younesia et al., Morinaga et al., Li, Schmidt et al., Sim et al., Vega et al., Cotter et al. and in Andreesen et al.

The first microorganism in the mixed culture according to any aspect of the present invention may be at least one acetogenic bacteria. The group of bacteria is one of the acetogenic anaerobic prokaryotes which can be used as a terminal electron acceptor of CO₂, thereby forming acetate or ethanol. Currently, 21 different genera of the acetogenic bacteria are known in the art (Drake et al., 2006), and these may also include some clostridia (Drake & Kusel, 2005). These bacteria are able to use carbon dioxide or carbon monoxide as a carbon with hydrogen as an energy source (Wood, 1991). Further, alcohols, aldehydes, carboxylic acids as well as numerous hexoses may also be used as a carbon source (Drake et al., 2004). The reductive pathway that leads to the formation of acetate is referred to as acetyl-CoA or Wood - Ljungdahl pathway.

According to any aspect of the present invention, the first microorganism may be an acetogenic microorganism selected from the group consisting of *Clostridium autothenogenum* DSMZ 19630, *Clostridium ragsdahlei* ATCC no. BAA-622, *Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum* ATCC 33266, *Clostridium formicoaceticum, Clostridium butyricum, Laktobacillus delbrukii, Propionibacterium acidoprprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium Ijungdahlii, Clostridium* ATCC 29797 and *Clostridium carboxidivorans.* In particular the strain ATCC BAA-624 of *Clostridium carboxidivorans* may be used. More in particular, the bacterial strain labelled "P7" and "P11" of *Clostridium carboxidivorans* as described for example in U.S. 2007/0275447 and U.S. 2008/0057554 may be used.

Another particularly suitable bacterium may be *Clostridium Ijungdahlii.* In particular, strains selected from the group consisting of *Clostridium Ijungdahlii* PETC, *Clostridium Ijungdahlii* ERI2, *Clostridium Ijungdahlii* COL and *Clostridium Ijungdahlii* O-52 may be selected for use as a first organism in the mixed culture of the present invention. These strains for example are described in WO 98/00558, WO 00/68407, ATCC 49587, ATCC 55988 and ATCC 55989.

In one example, the first microorganism in the mixed culture may be selected from the group consisting of ATCC BAA-1772 *Alkalibaculum Bacchi, Moor Ella sp.* HUC22-1, *Ijungdahlii Clostridium, Clostridium ragsdahlei,* and *Clostridium autoethanogenum.* The method of producing ethanol and/or acetate using this microorganism is disclosed for example in Saxena et al., Younesi et al., Sakai et al., Abrini et al. and the like.

In particular, the first microorganism may be functional in aerobic conditions in the presence of a second non-acetogenic microorganism.

According to any aspect of the present invention the acetate and/or ethanol, especially acetate, is reacted with the second microorganism in the mixed culture, to produce at least one substituted and/or unsubstituted organic compound. The second microorganism may be a microorganism wherein the glyoxylate cycle takes place to utilise the acetate and/or ethanol, especially the acetate.

In particular, the second microorganism may be non-acetogenic and may be yeast, microalgae and/or bacteria. More in particular, the second microorganism may be a genetically modified strain, wherein the yield of the substituted and/or unsubstituted organic compound is particularly optimized with regard to the genetic aspect of microorganism compared to the wild-type of the organism. In this context, it is in the second microorganism, which compared to its wild type, has an increased activity of at least a thioesterase. The phrase "in comparison to its wild type has an increased activity" means that the microorganism has been genetically modified so as to have this increased activity. A skilled person would understand that either an over-expression of a thioesterase in the second microorganism or an expression of an exogenous thioesterase may be applicable. In particular, the thioesterases may be selected from acyl-ACP thioesterases, EC 3.1.2.14 or EC 3.1.2.22, and acyl -CoA thioesterases, particularly EC 3.1.2.2, EC 3.1.2.18 , EC 3.1.2.19, EC 3.1.2.20 or EC 3.1.2.22 . Examples of second microorganisms that may be used in another example according to any aspect of the present invention are described in WO2010118410, WO2010075483, WO2008119082 and WO2007136762. These documents disclose these microorganisms with regard to these thioesterases.

The skilled person would understand the target molecule suitable for the second microorganism and the conditions to carry out the process.

More in particular, the second microorganism may be a yeast and the yeast may be an oleaginous yeast and/or may be selected from the group consisting of *Candida, Lipomyces, Paecilomyces, Rhodosporidhtm, Rhodotorula, Saccharomyces, Trichosporonaceae, Yarrowia* and *Cryptococcus.* In particular, the yeast may be selected from the group consisting of *Candida tropicalis, Candida lipolytica, Trichosporon cutaneum, Rhodosporidium toruloides, Paecilomyces varioti, Crytococcus curvatus,* and *Yarrowia lipolytica.*

The second microorganism may be microalgae and the microalgae may be *Crypthecodinium cohnii.*

In another example, the second microorganism may be a bacterium and the bacterium may be selected from the group consisting of *Staphylococcus sp., Streptococcus sp., Escherichia sp., Listeria sp., Ralstonia sp., Shewanella sp., Desulfomonas sp., Butyrivibrio sp., Eubacterium sp., Lactobacillus sp., Clostridium sp., Ruminococcus sp., Corynebacterium sp., Halomonas sp., and Propionibacterium sp.* In particular, the bacterium maybe selected from the group consisting of *Corynebacterium glutamicum, Halomonas boliviensis, Escherichia coli, Cupriavidus necator, Ralstonia eutropha* and *Pseudomonas putida.*

More in particular, the second microorganism may be selected from the group consisting of *Corynebacterium glutamicum, Crypthecodinum cohnii, Paecilomyces varioti, Halomonas boliviensis, Crytococcus curvatus, Escherichia coli, Cupriavidus necator, Pseudomonas putida* and *Yarrowia lipolytica.*

The method according to any aspect of the present invention may be especially advantageous as there is no extra step of removal of the intermediate product (ethanol and/or acetate) to react with the second microorganism for fermentation. All parts of the reaction take place in a single container allowing for a more efficient process and less wastage of intermediate products in the removal step.

According to any aspect of the present invention the acetate and/or ethanol, especially acetate, may be reacted with at least one second microorganism in the mixed culture, to produce at least one substituted and/or unsubstituted organic compound.

The substituted or unsubstituted organic compound may be selected from the group consisting of carboxylic acids, dicarboxylic acids, hydroxycarboxylic acids, carboxylic acid esters, hydroxycarboxylic acid esters, alcohols, aldehydes, ketones, amines, amino acids and the like. In particular, these organic compounds comprise 4 to 32, 6 to 20, or in particular 8 to 12 carbon atoms. In particular, the organic compound may be 3-hydroxy isobutyric acid, acetate, methanoate, fatty acid (C₈ to C₁₂), succinate and the like.

More in particular, the organic compounds produced by the mixed culture of the present invention are carboxylic acids, hydroxycarboxylic acids and/or carboxylic acid esters. The concentration of the final product using the method of the present invention may exceed the intermediate acetate or ethanol during the method of the present invention.

The method according to the present invention may further comprise a step of separating and/or purifying the substituted or unsubstituted organic compound. The method used for separation and/or purifying may be any method known in the art. In particular, the step of separating the organic compound comprises removal of the microorganisms by sedimentation, centrifugation or filtration of the medium.

According to a further aspect of the present invention, there is provided a use of the mixed culture according to the present invention for preparing a substituted or unsubstituted organic compound.

### BRIEF DESCRIPTION OF THE FIGURES

No figures.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### Production of 3-hydroxy isobutyric acid (3HIB) from synthesis gas using mixed production

Steps 1 to 3 of Example 1 in EP2602329A1 is followed to produce a *Yarrowia lipolytica* cell H222-41 with attenuated activity of 3-hydroxy isobutyric acid dehydrogenase. This cell is labelled as H22-41 Δ3HIBDH (ura)-8.

For the biotransformation of synthesis gas (0.6 % O₂, 33 % CO₂, 66.4 % H₂) to form 3HIB, *Clostridium Ijungdahlii* (DSMZ13528) and *Yarrowia lipolytica* H222-41 Δ3HIBDH (ura)-8 are used. Both strains are cultured separately. For C. *Ijungdahlii* the assays and experiments are carried out in anaerobic conditions unless otherwise specified.

*Clostridium Ijungdahlii* is adapted to grow in an environment comprising oxygen. 5 ml of a *Clostridium Ijungdahlii* culture is combined with 500ml of ATCC1754 medium (pH 6.0, 20 g/L MES, 1 g/L yeast extract, 0.8 g/L NaCl , 1 g/L NH₄Cl, 0.1 g/L KCI, 0.1 g/L KH₂PO₄, 0.2 g/L MgSO₄.7H₂O, 0.02 g/L CaCl₂ x 2H₂O, 20 mg/L nitrilotriacetic acid, 10 mg/L MnSO₄ x H₂O, 8 mg/L (NH₄)₂Fe(SO₄)₂ x 6 H₂O, CoCl₂ x 6 H₂O, 2 mg/L ZnSO₄ x 7 H₂O, 0.2 mg/L CuCl₂ x 2 H₂O, 0.2 mg/L Na₂MoO₄ x 2 H₂O, 0.2 mg/L NiCl₂ x 6 H₂O, 0.2 mg/L Na₂SeO₄, 0.2 mg/L Na₂WO₄ x 2 H₂O, 20 µg/L d-Biotin, 20 µg/L folic acid, 100 µg/L pyridoxine HCl, 50 µg/L thiamine-HCl x H₂O, 50 µg/L riboflavin , 50 µg/L nicotinic acid , 50 µg/L Ca-pantothenate, 1 µg/L vitamin B₁₂, 50 µg/L p-aminobenzoate, 50 µg/L lipoic acid, about 67.5 mg/L NaOH (to adjust the pH) and 400 mg/L L-cysteine-HCl). The culture is gassed with 1 L/h (66.85 % H₂, 33 % CO₂, and 0.15 % O₂) at 37 °C, 100 rpm for 140 h and cultivated in pressure-resistant glass bottles that are hermetically sealed with a butyl rubber stopper.

Subsequently, this culture is inoculated with a starting OD of 0.2 in 200 mL of ATCC1754 medium and cultured for 2-3 days with gassing of 1 L/h of gas mixture (66.4 % H₂, 33 % CO₂, and 0.6 % O₂). From this first culture, new cryo cultures of the O₂-adapted C. *Ijungdahlii* are formed. 4 ml of the culture broth is mixed with 1 mL of 25 % DMSO solution and frozen at -80 °C for future use.

5 mL of a cryo culture of the O₂-adapted *C. Ijungdahlii* is then added to 200 mL of ATCC1754 medium and cultured with gassing of 1 L/h of synthesis gas (66.1 % H₂, 33.3 % CO₂, and 0.6 % O₂) at 37 °C and 100 rpm for 3-6 days.

*Yarrowia lipolytica* cell H222-41 Δ3HIBDH (ura)-8, is spread and incubated on a YPD plate (10 g/L glucose, 10 g/L yeast extract, 20 g/L peptone and 15 g/L agar, pH 5.4) at 28 °C for 20 h. Then, a shake flask (1000 mL non-baffled) is filled with 100 ml of YPD medium (10 g/L glucose, 10 g/L yeast extract, 20 g/L peptone, and 3 drops of antifoam Delamex) and inoculated with a loop full of the cell material of the YPD plate. Incubation is carried out aerobically for 20h at 28 °C and at 200 rpm.

For the mixing phase of production, the cells are harvested from both the cultures and washed with two growth production buffers (pH: 7.0, 0.5 g/l sodium acetate, 400 mg/l L-cysteine hydrochloride) separately. The cells are then transferred into separate Falcon tubes and centrifuged at 5100rpm for 10 min. The supernatant is discarded and the cells in each tube are washed in 10 ml of production buffer. After centrifugation at 5100rpm for 10 min, the cells are resuspended in 5mL production buffer.

The cell masses of the two strains are combined in a pressure-tight, sterile glass bottle (volume 500 mL) filled with 200 mL of production buffer and the mixed production phase starts. The mixed production takes place under 1 L/h fumigation with synthesis gas (66.4 % H₂, 33 % CO₂, and 0.6 % O₂) and incubated at 30 °C. During the production phase, 5 mL sample volume is taken daily to determine the optical density, the pH, and various analytes by NMR. This shows the formation of ethanol and 3HIB. These measurements are taken using semi-quantitative 1 H-NMR spectroscopy of the sterile and filtered supernatant from the mixed production. The samples are in accordance with phosphate buffer diluted (in stated D₂O) and measured with water suppression. As an internal quantification standard, sodium trimethylsilylpropionate (TSP) is used.

### Example 2

### Culture of C. Ijungdahlii in the presence of O₂ (0.15 %) and synthesis gas

Wild-type strain *Clostridium Ijungdahlii* (DSMZ13528) was cultivated autotrophically in a complex medium (1 g/l NH₄Cl, 0.1 g/l KCI, 0.2 g/l MgSO₄ x 7H₂O, 0.8 g/l NaCl, 0.1 g/l KH₂PO₄, 20 mg/l CaCl₂ x 2 H₂O, 20 g/l MES, 1 g/l yeast extract, 0.4 g/l L-Cysteine-HCl, 20 mg/l nitrilotriacetic acid, 10 mg/l MnSO₄ x H₂O, 8 mg/l (NH₄)₂Fe(SO₄)₂ x 6 H₂O, 2 mg/l CoCl₂ x 6 H₂O, 2 mg/l ZnSO₄ x 7 H₂O, 0.2 mg/l CuCl₂ x 2 H₂O, 0.2 mg/l Na₂MoO₄ x 2 H₂O, 0.2 mg/l NiCl₂ x 6 H₂O, 0.2 mg/l Na₂SeO₄, 0.2 mg/l Na₂WO₄ x 2 H₂O, 20 µg/l d-Biotin, 20 µg/l folic acid, 100 µg/l Pyridoxin-HCl, 50 µg/l Thiamine-HCl x H₂O. 50 µg/l Riboflavin, 50 µg/l nicotinic acid, 50 µg/l Ca-Pantothenate, 1 µg/l Vitamin B12, 50 µg/l p-Aminobenzoate, 50 µg/l lipoic acid).

Identical autotrophic cultivations were carried out in 1I-septum bottles with 500 ml of complex medium placed in an open water bath shaker Innova 3100 by New Brunswick Scientific and shaking at speed of 150 min⁻¹. The gas trapped in the medium was removed by a frit with a pore size of 10 microns, which was mounted in the center of the reactors. The cultivations were performed at 37 °C without pH control. The reactors were purged with a pre-mixed, non-explosive gas mixture of the composition of 66.75 % H₂, 33 % CO₂ and 0.15 % O₂ at atmospheric pressure with an aeration rate of 1 b l/h (0.033 vvm).

The reactors with the entire content were inoculated with of 5 ml of DMSO (5 %) to produce a cryo-culture. The bacterial culture in cryo was cultured autotrophically in the presence of synthesis gas mixture of 67 % H₂, 33 % CO₂ in the absence of O₂ in the abovementioned complex medium.

During the process of sampling, 5 ml of each sample was taken to determine the OD₆₀₀, pH and product spectrum of the sample. The determination of the product concentration was performed using quantitative 1 H- NMR spectroscopy. Sodium trimethylsilylpropionate (T(M) SP) served as an internal quantification standard.

Surprisingly, it was found that, the cryogenic acetogenic bacterial cells C. *Ijungdahlii* could grow in the presence of 0.15 % O₂ and form C. *Ijungdahlii* typical products (acetate, ethanol) from the synthesis gas. An attempt to repeat this result was not successful as shown in Table 1 as experiments 2-6. In experiments 2-6, the bacteria cells did not manage to grow in the presence of 0.15 % O₂ in the synthesis gas from the cryo- culture. The cellular material from experiment 1 was then used for further experiments to study O₂-containing synthesis gas.

In one of the further experiments, the cellular material from experiment 1 was used as a sample for sequencing of the bacterial genome to identify possible changes in the genome of C. *Ijungdahlii,* in the presence of O₂. The sequencing results showed no changes in the genome of C. *Ijungdahlii* which were attributed to the presence of O₂. However, contamination of the bacterial culture (~ 70 % of the genomic DNA based on the sequencing results) with the skin germ *Propionibacterium* acnes was identified. The cell material from the cryo-cultures used in experiment 1 was compared with that used for experiments 2-6 by using sequencing and *Propionibacterium -* specific gene probe. The results showed that in the cryo-culture used in experiments 2-6 no *Propionibacterium* acnes were present method (detection limit: 0.1 %). In cryo- cultures which were used for experiment 1, there was *Propionibacterium* acnes and 1-2 % of C. *Ijungdahlii* found.

*Propionibacterium* acnes are aero -tolerant, anaerobic organisms that can consume O₂ in their culture during metabolism. The unintentionally encountered contamination of the culture of C. *Ijungdahlii* with *Propionibacterium* acnes has thus led to the reduction in O₂ content in the liquid phase and thus creation of an O₂ free environment for the growth of strictly anaerobic organism C. *Ijungdahlii.*

**Table 1. Results of Example 2.**

| | | | | NMR | | |
|---|---|---|---|---|---|---|
| Experiment | Zeit, h | pH | OD600 | n-Propansäure, mg/L | Ethanol, mg/L | Acetat, mg/L |
| **1** | 0,0 | 5,73 | 0,042 | | | |
| | 67,1 | 5,73 | 0,084 | 0 | 9 | 296 |
| | 93,3 | 5,62 | 0,173 | | | |
| | 118,8 | 5,33 | 0,263 | 0 | 362 | 4495 |
| | 142,8 | 5,01 | 0,406 | | | |
| **2** | 0,0 | 6,02 | 0,034 | | | |
| | 24,0 | 5,91 | 0,031 | | | |
| | 41,0 | 5,96 | 0,027 | | | |
| | 68,3 | 5,94 | 0,027 | n.q. | n.q. | 15 |
| | 140,4 | 5,94 | 0,074 | | | |
| | 160,7 | 5,83 | 0,065 | | | |
| **3** | 0,0 | 6,02 | 0,031 | | | |
| | 24,0 | 5,97 | 0,030 | | | |
| | 41,0 | 5,95 | 0,026 | | | |
| | 68,3 | 5,90 | 0,027 | 0 | n.q. | 20 |
| | 140,4 | 5,94 | 0,081 | | | |
| | 160,7 | 5,88 | 0,084 | | | |
| **4** | 0,0 | 5,92 | 0,079 | keine Daten | | |
| | 21,0 | 5,92 | 0,065 | | | |
| | 42,3 | 5,82 | 0,064 | | | |
| | 66,5 | 5,85 | 0,059 | | | |
| | 88,7 | 5,91 | 0,057 | | | |
| **5** | 0,0 | 5,95 | 0,076 | keine Daten | | |
| | 21,0 | 5,95 | 0,061 | | | |
| | 42,3 | 5,81 | 0,060 | | | |
| | 66,5 | 5,85 | 0,055 | | | |
| | 88,7 | 5,89 | 0,053 | | | |
| **6** | 0,0 | 5,96 | 0,071 | keine Daten | | |
| | 21,0 | 5,95 | 0,065 | | | |
| | 42,3 | 5,83 | 0,064 | | | |
| | 66,5 | 5,86 | 0,057 | | | |
| | 88,7 | 5,90 | 0,053 | | | |

### Example 3

### Co-fermentation of Clostridium Ijungdahlii and E. coli for producing acetate, methanoate and fatty acids

For the biotransformation of synthesis gas to fatty acids (C₈ to C₁₂), a co-production phase of *Clostridium Ijungdahlii* and a plasmid-bearing *Escherichia coli* as described below was used.

The bacterium *Clostridium Ijungdahlii* is a homoacetogenic bacteria (i.e. strictly anaerobic) that formed acetate and methanoate from a feed-through gas phase of H₂ and CO₂. These products of acetate and methanoate were then taken up by *E. coli* from the aqueous phase and converted to fatty acids.

For the cultivation of these cells, pressure-resistant glass bottles that could be sealed airtight with a butyl rubber stopper were used. All culture steps, in which C. *Ijungdahlii* cells were involved were carried out in anaerobic conditions. The plasmid-carrying *E. coli* strain used was *E. coli* strain JW5020-1 ΔfadE pJ294[Ptac-ChFATB2_optEC]. Here the original strain *E. coli* JW5020-1 originated from the Keio collection (Baba et al., 2006). The construction of the introduced plasmid pJ294[Ptac-ChFATB2_optEC] is described in Example 2 of DE102011110945A1, the ΔfadE deletion as well as the transformation of *E. coli* strain origin was carried out analogously to Example 4 of DE102011110945A1.

For the cell culture of C. *Ijungdahlii,* 5 ml of cryoculture was anaerobically grown in 50 ml of medium (ATCC1754 medium: pH 6.0, 20 g/l MES; 1 g/l yeast extract, 0.8 g/l NaCl; 1 g/l NH₄Cl; 0.1 g/l KCI; 0.1 g/l KH₂PO₄; 0.2 g/l MgSO₄ x 7 H₂O; 0.02 g/l CaCl₂ x 2 H₂O; 20 mg/l nitrilotriacetic acid; 10 mg/l MnSO₄ x H₂O; 8 mg/L (NH₄)₂Fe(SO₄)₂ x 6 H₂O; 2 mg/l CoCl₂ x 6 H₂O; 2 mg/l ZnSO₄ x 7 H₂O; 0.2 mg/l CuCl₂ x 2 H₂O; 0.2 mg/l Na₂MoO₄ x 2 H₂O; 0.2 mg/l NiCl₂ x 6 H₂O; 0.2 mg/l Na₂SeO₄; 0.2 mg/l Na₂WO₄ x 2 H₂O; 20 µg/L d-Biotin; 20 µg/l folic acid; 100 µg/l Pyridoxine-HCl; 50 µg/l Thiamine-HCl x H₂O; 50 µg/l Riboflavin; 50 µg/l nicotinic acid; 50 µg/l Ca-Pantothenate; 1 µg/l Vitamin B₁₂; 50 µg/l p-Aminobenzoate; 50 µg/l lipoic acid; ca. 67.5 mg/l NaOH; 400 mg/l L-Cysteine-hydrochloride). Cultivation was carried out in a 250 ml glass bottle to a pressure of 1 bar with a premixed gas mixture of composition 67 % H₂, 33 % CO₂ at 37 °C and 150 rpm for 74 hours in an open water-bath shaker. The culture was then centrifuged and the cells inoculated in 200 ml of fully anaerobic ATCC1754 medium. The cultivation was carried out chemolithoautotrophically in a pressure-resistant 500-ml glass bottle with a premixed gas mixture composed of 67 % H₂, 33 % CO₂ in an open water bath shaker at 37 °C, 150 rpm and an aeration of 3 l/h for 66h. The gas entry into the medium was carried out by a frit with a pore size of 10µm and mounted in the center of the reactor of the sparger. The cells were centrifuged, washed with 10 ml of washing buffer (0.7 g/l KOH for 2 h with a premixed gas mixture of composition 66 % H₂, 33 % CO₂) and washed again by centrifugation.

For the cell culture of *E. coli* 200 ml of medium was inoculated (pH 7.0; 6.79 g/l Na₂HPO₄; 2.6 g/l (NH₄)₂SO₄; 3 g/l KH₂PO₄; 0.5 g/l NaCl; 2 g/l NH₄Cl; 2 ml/l 1 M MgSO₄ x 7 H₂O; 39.84 ml/l Na-Acetate (123 g/l, pH 7.0); 0.13 ml/l 25 % HCl; 1.91 mg/l MnCl₂ x 7 H₂O; 1.87 mg/l ZnSO₄ x 7 H₂O; 0.84 mg/l Na-EDTA x 2 H₂O; 0.3 mg/l H₃BO₃; 0.25 mg/l Na₂MoO₄ x 2 H₂O; 4.7 mg/l CaCl₂ x 2 H₂O; 17.8 mg/l FeSO₄ x 7 H₂O; 0.15 mg/l CuCl₂ x 2 H₂O; 100 mg/l Ampicillin) with a single colony from an agar plate. The cultivation was carried out in a pressure-tight 500 ml glass bottle with a premixed gas mixture composed of 90 % H₂, 6 % CO₂, 4 % O₂ in an open water bath shaker at 37 °C, 150 rpm and an aeration of 1 l/h for 115 h and the gas trapped in the medium was carried out using a frit with a pore size of 10 µm. These frits were mounted in the center of the reactors on a sparger. 200 µl of IPTG solution (1 M) was added after 72 h. The cells were then centrifuged and washed with 10 ml washing buffer (0.5 g/l sodium acetate, pH 7.0), and centrifuged again.

For the joint production phase the washed cells from both growth cultures (*E.coli* at OD₆₀₀ of 1.0 and C. *Ijungdahlii* at OD₆₀₀ of 0.2) were grown in 200 ml of production buffer (pH: 7.0, 0.5 g/l sodium acetate, 400 mg/l L-cysteine hydrochloride). The cultivation was carried out in a pressure-tight 500 ml glass bottle with a premixed gas mixture composed of 65 % H₂, 33 % CO₂, 2 % O₂ in an open water bath shaker at 37 °C, 150 rpm with an aeration of 1 l/h for 18 h. The gas trapped in the medium was carried out by a frit with a pore size of 10 µm, which was mounted in the center of the reactors of a sparger.

For sampling, 5 ml of each sample was taken for determination of OD₆₀₀, pH, and the product spectrum. The determination of the product concentration was performed by semi-quantitative 1 H-NMR spectroscopy. Standard sodium trimethylsilylpropionate served (T(M)SP) served as an internal quantification.

After the cultivation period, the results showed that there was an increase in acetate from 330 mg/l to 378 mg/l and methanoate from 14 g/l to 36 g/l. In addition, fatty acid (C₈ to C₁₂) concentration increased from 0 mg/l to 7 mg/l.

### Example 4

### Succinate production from synthesis gas

For the biotransformation of synthesis gas to succinate co-production phase of *Clostridium Ijungdahlii* and plasmid-carrying *Escherichia coli* was used. The homoacetogenic bacterium *Clostridium Ijungdahlii* converted H₂ and CO₂ from the gas phase to and made acetate and methanoate which were then taken up by *E. coli* from the aqueous phase and converted to succinate.

For the cultivation of the bacteria, pressure-resistant glass bottles that can be sealed airtight with a butyl rubber stopper were used. All culture steps, in which C. *Ijungdahlii* cells were involved were carried out under anaerobic conditions. The plasmid-carrying *E. coli* strain, *E. coli* strain JW5020-1 ΔfadE pJ294[Ptac-ChFATB2_optEC] was used. The origin strain *E. coli* JW5020-1 originates from the Keio collection (Baba et al., 2006). The construction of the introduced plasmid pJ294[Ptac-ChFATB2_optEC] is described in Example 2 of DE102011 110945A1, the ΔfadE deletion as well as the transformation of *E. coli* strain origin was carried out analogously to Example 4 of DE102011110945A1.

For the cell culture of C. *Ijungdahlii,* 2 ml of cryoculture was anaerobically grown in 200 ml of medium (ATCC1754 medium: pH 6.0, 20 g/l MES; 1 g/l yeast extract, 0.8 g/l NaCl; 1 g/l NH₄Cl; 0.1 g/l KCI; 0.1 g/l KH₂PO₄; 0.2 g/l MgSO₄ x 7 H₂O; 0.02 g/l CaCl₂ x 2 H₂O; 20 mg/l nitrilotriacetic acid; 10 mg/l MnSO₄ x H₂O; 8 mg/L (NH₄)₂Fe(SO₄)₂ x 6 H₂O; 2 mg/l CoCl₂ x 6 H₂O; 2 mg/l ZnSO₄ x 7 H₂O; 0.2 mg/l CuCl₂ x 2 H₂O; 0.2 mg/l Na₂MoO₄ x 2 H₂O; 0.2 mg/l NiCl₂ x 6 H₂O; 0.2 mg/l Na₂SeO₄; 0.2 mg/l Na₂WO₄ x 2 H₂O; 20 µg/L d-Biotin; 20 µg/l folic acid; 100 µg/l Pyridoxine-HCl; 50 µg/l Thiamine-HCl x H₂O; 50 µg/l Riboflavin; 50 µg/l nicotinic acid; 50 µg/l Ca-Pantothenate; 1 µg/l Vitamin B₁₂; 50 µg/l p-Aminobenzoate; 50 µg/l lipoic acid; ca. 67.5 mg/l NaOH; 400 mg/l L-Cysteine-hydrochloride). Cultivation was carried out in a 500 ml glass bottle to a pressure of 1 bar with a premixed gas mixture of composition 67 % H₂, 33 % CO₂ at 37 °C and 150 rpm for 111 hours in an open water-bath shaker. The culture was then centrifuged and washed with 10ml of wash buffer (pH 6.2, 0.1 g/l Na-acetate, 100 mg/l L-cysteine hydrochloride).

For the cell culture of *E. coli* 200 ml of medium was inoculated (pH 7.0; 6.79 g/L Na₂HPO₄; 2.6 g/l (NH₄)₂SO₄; 3 g/l KH₂PO₄; 0.5 g/l NaCl; 2 g/l NH₄Cl; 2 ml/l 1 M MgSO₄ x 7 H₂O; 39.84 ml/l Na-Acetate (123 g/l, pH 7.0); 0.13 ml/l 25 % HCl; 1.91 mg/l MnCl₂ x 7 H₂O; 1.87 mg/l ZnSO₄ x 7 H₂O; 0.84 mg/l Na-EDTA x 2 H₂O; 0.3 mg/l H₃BO₃; 0.25 mg/l Na₂MoO₄ x 2 H₂O; 4.7 mg/l CaCl₂ x 2 H₂O; 17.8 mg/l FeSO₄ x 7 H₂O; 0.15 mg/l CuCl₂ x 2 H₂O; 100 mg/l Ampicillin) with a single colony from an agar plate and covered. The cultivation was carried out in a pressure-tight 500 ml glass bottle with a premixed gas mixture composed of 80 % N₂ 10 % H₂, 6 % CO₂, 4 % O₂ in an open water bath shaker at 37 °C, 150 rpm and an aeration of 0.7 l/h for 160 h and the gas trapped in the medium was carried out using a frit with a pore size of 10 µm. These frits were mounted in the center of the reactors on a sparger. 200 µl of IPTG solution (1 M) was added after 115 h. The cells were then centrifuged and washed with 10 ml washing buffer (pH 7.1, 0.1 g/l Na-acetate, 100 mg/l L-cysteine hydrochloride) and centrifuged again.

For the joint production phase the washed cells from both growth cultures (*E.coli* at OD₆₀₀ of 0.6 and C. *Ijungdahlii* at OD₆₀₀ of 0.2) were grown in 200 ml of production buffer (pH: 6.5, 0.1 g/l sodium acetate, 100 mg/l L-cysteine hydrochloride). The cultivation was carried out in a pressure-tight 500 ml glass bottle with a premixed gas mixture composed of 65 % H₂, 33 % CO₂, 2 % O₂ in an open water bath shaker at 37 °C, 150 rpm for 113 h. The gas phase was replaced every 24 h with fresh gas of the same composition. After 14 h were 40 ppm of L-cysteine hydrochloride was added and after 38 h, a further 100 ppm of L-cysteine hydrochloride was added.

For sampling, 5 ml of each sample was taken for determination of OD₆₀₀, pH, and the product spectrum. The determination of the product concentration was performed by semi-quantitative 1 H-NMR spectroscopy. Standard sodium trimethylsilylpropionate served (T(M)SP) served as an internal quantification.

After the cultivation period, the results showed that there was an increase in acetate from 77 mg/l to 101 mg/l and methanoate from 0 mg/l to 174 g/l. In addition, succinate concentration increased from 0 mg/l to 40 mg/l.

### REFERENCES

1. Demler M, et al. Reaction engineering analysis of hydrogenotrophic production of acetic acid by Acetobacterium woodii. Biotechnol Bioeng . February 2011, 108 (2):470-4.
2. Reed, TB, 1981, Biomass gasification: principles and technology, Noves Data Corporation, Park Ridge, NJ.
3. Younesia et al. Ethanol and acetate production from synthesis gas via fermentation processes using anaerobic bacterium, Clostridium Ijungdahlii. Biochemical Engineering Journal, Volume 27, Issue 2, Pages 110-119.
4. Morinaga et al. The production of acetic acid from carbon dioxide and hydrogen by an anaerobic bacterium. Journal of Biotechnology, Volume 1, Issue 2, Pages 187-194.
5. Li, Production of acetic acid from synthesis gas with mixed acetogenic microorganisms, ISSN 0493644938.
6. Schmidt et al. Production of acetic acid from hydrogen and carbon dioxide by Clostridium species ATCC 2979th Chemical Engineering Communications, 45:1-6 , 61-73.
7. Sim et al. Optimization of acetic acid production from synthesis gas by chemolithotrophic bacterium - Clostridium aceticum using a statistical approach. Bioresour Technol. 2008 May ; 99 (8):2724 -35
8. Vega et al. Study of gaseous substrates fermentations CO conversion to acetate 1 batch culture or 2 continuous culture. Biotechnology and Bioengineering Volume 34, Issue 6, pages 774, or 785, September 1989.
9. Cotter et al. Ethanol and acetate production by Clostridium Ijungdahlii and Clostridium autoethanogenum using resting cells. Bioprocess and Biosystems Engineering (2009), 32 (3), 369-380.
10. Andreesen et al. Fermentation of glucose, fructose, and xylose by Clostridium thermoaceticum. Effect of metals on growth yield, enzymes, and the synthesis of acetate from carbon dioxide. Journal of Bacteriology (1973), 114 (2), 743-51.
11. Drake et al., 2006
12. Drake & Kusel, 2005
13. Wood, 1991
14. Drake et al., 2004.
15. Saxena et al. Effect of trace metals on ethanol production from synthesis gas by the ethanologenic acetogen Clostridium ragsdalei. Journal of Industrial Microbiology & Biotechnology Volume 38, Number 4 (2011), 513-521,
16. Younesi et al. Ethanol and acetate production from synthesis gas via fermentation processes using anaerobic bacteriumClostridium Ijungdahlii. Biochemical Engineering Journal Volume 27, Issue 2, 15 December 2005, Pages 110-119,
17. Sakai et al. Ethanol production from H2 and CO2 by a newly isolated thermophilic bacterium, Moor Ella sp. HUC22-1. Biotechnology Letters Volume 26, Number 20 (2004), 1607-1612 and
18. Abrini et al. Autoethanogenum Clostridium sp. nov., to anaerobic bacterium did Produces ethanol from carbon monoxide. Archives of Microbiology Volume 161, Number 4 (1994), 345-351.
19. Baba et al. , 2006
20. WO2013167663, WO2013036147, WO200068407, WO2012024522, WO9800558, WO2000014052, WO2010115054, WO 98/00558, WO 00/68407, WO2010118410, WO2010075483, WO2008119082, WO2007136762, U.S. 2007/0275447, DE102011110945A1 and U.S. 2008/0057554.

## Claims

1. A mixed culture of a first and second microorganism in an aqueous medium, wherein
- the first microorganism is an acetogenic microorganism capable of converting a carbon source comprising CO and/or CO₂ to acetate and/or ethanol;
- the second microorganism is a non-acetogenic microorganism capable of metabolising acetate and/or ethanol; and
- the first and second microorganisms are in a homogenous mixture.

2. The culture according to claim 1, wherein second microorganism is selected from the group consisting of aerotolerant microorganisms, facultative anaerobes and aerobic microorganisms.

3. The culture according to either claim 1 or 2, wherein the aqueous medium comprises oxygen.

4. The culture according to claim 3, wherein the aqueous medium comprises oxygen at a range of 0.1 to 1.5mg/l.

5. The culture according to any of the preceding claims, wherein the carbon source comprises at least 50 % by weight of CO and/or CO₂.

6. The culture according to any of the preceding claims, wherein the first microorganism is selected from the group consisting of *Clostridium autothenogenum DSMZ 19630, Clostridium ragsdahlei A TCC no. BAA-622, Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum A TCC 33266, Clostridium formicoaceticum, Clostridium butyricum, Laktobacillus delbrukii, Propionibacterium acidoprprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica,*
*Eubacterium limosum, Peptostreptococcus productus, Clostridium Ijungdahlii, Clostridium A TCC* 29797 and *Clostridium carboxidivorans.*

7. The culture according to any one of the preceding claims, wherein the second microorganism is at least a yeast, microalgae and/or bacteria and wherein
- the yeast is selected from the group consisting of *Candida tropicalis, Candida lipolytica, Trichosporon cutaneum, Rhodosporidium toruloides, Paecilomyces varioti, Crytococcus curvatus,* and *Yarrowia lipolytica;*
- the microalgae is *Crypthecodinum cohnii;* and/or
- the bacteria is selected from the group consisting of *Corynebacterium glutamicum, Halomonas boliviensis, Escherichia coli, Cupriavidus necator, Ralstonia eutropha* and *Pseudomonas putida.*

8. The culture according to any one of the preceding claims, wherein the acetate and/or ethanol is metabolised to at least one substituted or unsubstituted organic compound.

9. A method of producing at least one substituted or unsubstituted organic compound, the method comprising contacting a mixed culture of a first and second microorganism in a homogenous mixture in an aqueous medium with a carbon source, wherein
- the first microorganism is an acetogenic microorganism capable of converting the carbon source to acetate and/or ethanol; and
- the second microorganism is a non-acetogenic microorganism capable of metabolising acetate and/or ethanol to the substituted or unsubstituted organic compound.

10. The method according to claim 9, wherein the second microorganism is at least a yeast, microalgae and/or bacteria and wherein
- the yeast is selected from the group consisting of *Candida tropicalis, Candida lipolytica, Trichosporon cutaneum, Rhodosporidium toruloides, Paecilomyces varioti, Crytococcus curvatus,* and *Yarrowia lipolytica;*
- the microalgae is *Crypthecodinum cohnii;* and/or
- the bacteria is selected from the group consisting of *Corynebacterium glutamicum, Halomonas boliviensis, Escherichia coli, Cupriavidus necator, Ralstonia eutropha* and *Pseudomonas putida.*

11. The method according to either claim 9 or 10, wherein the aqueous medium comprises oxygen and a carbon source comprising CO and/or CO₂.

12. The method according to claim 11, wherein the oxygen and the carbon source comprising CO and/or CO₂ is provided to the aqueous medium in a continuous gas flow.

13. The method according to either claim 11 or 12, wherein the amount of oxygen in the aqueous medium is in a range of 0.1 to 1.5mg/l.

14. The method according to either claim 12 or 13, wherein the continuous gas flow has a concentration of oxygen at a range of 0.01 to 10 % by weight.

15. The method according to any one of claims 9 to 14, wherein the first microorganism is selected from the group consisting of *Clostridium autothenogenum* DSMZ 19630, *Clostridium ragsdahlei* ATCC no. BAA-622, *Clostridium autoethanogenum, Moorella sp* HUC22-1, *Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum* ATCC 33266, *Clostridium formicoaceticum, Clostridium butyricum, Laktobacillus delbrukii, Propionibacterium acidoprprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium Ijungdahlii, Clostridium* ATCC 29797 and *Clostridium carboxidivorans.*

16. The method according to any one of claims 9 to 15, wherein the second microorganism is at least a yeast, microalgae and/or bacteria and wherein
- the yeast is *Candida tropicalis, Candida lipolytica, Trichosporon cutaneum, Rhodosporidium toruloides, Paecilomyces varioti, Crytococcus curvatus,* and *Yarrowia lipolytica;*
- the microalgae is *Crypthecodinum cohnii;* and/or
- the bacteria is selected from the group consisting of *Corynebacterium glutamicum, Halomonas boliviensis, Escherichia coli, Cupriavidus necator, Ralstonia eutropha* and *Pseudomonas putida.*

17. The method according to any one of claims 9 to 16, wherein the substituted or unsubstituted organic compound is selected from the group consisting of carboxylic acids, dicarboxylic acids, hydroxycarboxylic acids, carboxylic acid esters, hydroxycarboxylic acid esters, alcohols, aldehydes, ketones, amines and amino acids.

18. Use of the mixed culture according to any one of claims 1 to 8 for preparing a substituted or unsubstituted organic compound.
